(19) Europäisches Patentamt
European Patent Office
Office européen des brevets

(11) Numéro de publication: **0 300 107**
**A1**

(12) **DEMANDE DE BREVET EUROPEEN**

(21) Numéro de dépôt: 87401722.1

(22) Date de dépôt: 23.07.87

(51) Int. Cl.[4]: **C07D 491/04 , C07D 519/00 , C08G 65/32 , A61K 31/47 , A61K 31/765 , A61K 47/00 , //(C07D491/04,317:00,221:00), (C07D519/00,491:00,491:00)**

Claims for the following Contracting States: ES + AT.

(43) Date de publication de la demande:
**25.01.89 Bulletin 89/04**

(84) Etats contractants désignés:
**AT BE CH DE ES GB IT LI LU NL SE**

(71) Demandeur: **VETOQUINOL S.A.**
**Magny-Vernois**
**F-70200 Lure(FR)**

(72) Inventeur: **Frechin, Etienne**
**1bis, rue du Vergerot**
**F-70200 Lure(FR)**
Inventeur: **Colin, Jean-Luc**
**25, place Charles de Gaulle**
**F-70200 Lure(FR)**

(74) Mandataire: **Kedinger, Jean-Paul et al**
**c/o Cabinet Malemont 42, avenue du Président Wilson**
**F-75116 Paris(FR)**

(54) **Nouveaux dérivés ester, thioester et amide de l'acide oxolinique, leur procédé de préparation et leur application en thérapeutique.**

(57) Ester, thioester ou amide obtenu par réaction entre l'acide oxolinique ou un dérivé réactif de celui-ci au niveau du groupe carboxyle et un groupe terminal OH, SH ou $NH_2$ d'un homopolymère ou un copolymère alterné, séquencé ou statistique, comportant d'une part, une moyenne de 2 à 50 motifs identiques ou différents de structure $-(CHR'CHR''O)-$ où $R'$ et $R''$ représentent indépendemment l'un de l'autre un atome d'hydrogène ou un groupe alkyle en $C_1$-$C_4$ et d'autre part, un premier groupe terminal OH, SH ou $NH_2$ et un deuxième groupe terminal OR où R = H, alkyle en $C_1$-$C_4$ éventuellement substitué par OH ou reste éther cyclique saturé à 5 ou 6 chaînons.

Cet ester, thioester ou amide est utile en tant qu'agent antibiotique.

EP 0 300 107 A1

## Nouveaux dérivés ester, thioester et amide de l'acide oxolinique, leur procédé de préparation et leur application en thérapeutique

La présente invention a pour objet de nouveaux dérivés de l'acide oxolinique, comprenant dans leur molécule une liaison ester, thioester ou amide ; elle a également pour objet le procédé de préparation de ces dérivés et leur application en thérapeutique.

L'acide oxolinique est un antibiotique de synthèse intéressant pour son spectre d'acitivité, sa bonne résorption par voie orale et sa bonne tolérance générale. Dans le souci d'améliorer aussi bien la solubilité de cet acide que sa concentration tissulaire et sa durée d'action, la Demanderesse a cherché à préparer des dérivés susceptibles de donner davantage de satisfaction sur ces deux points. Elle a ainsi mis en évidence que la condensation du groupe carboxyle en position 3 de l'acide oxolinique, avec un groupe terminal OH, SH ou $NH_2$ d'un homopolymère ou d'un copolymère alterné, séquencé ou statistique, comportant d'une part une moyenne de 2 à 50, de préférence 2 à 10, motifs identiques ou différents de structure $-(CHR'CHR''O)-$ où $R'$ et $R''$ représentent indépendamment l'un de l'autre un atome d'hydrogène ou un groupe alkyle en $C_1$-$C_4$, et d'autre part, un premier groupe terminal OH, SH ou $NH_2$ et un deuxième groupe terminal OR où R = H, alkyle en $C_1$-$C_4$ éventuellement substitué par un radical hydroxyle, ou reste éther cyclique saturé à 5 ou 6 chaînons, en vue de la formation d'une liaison ester, thioester ou amide, conduisait à de nouveaux dérivés de cet acide, faisant preuve des améliorations recherchées.

La présente invention a ainsi pour objet dans sa conception la plus générale, les esters, thioesters et amides obtenus par réaction entre le groupe carboxyle en position 3 de l'acide oxolinique ou d'un dérivé réactif de celui-ci, et un groupe terminal OH, SH ou $NH_2$ d'un homopolymère ou copolymère tel que défini ci-dessus.

Ces esters, thioesters et amides selon l'invention comprennent notamment ceux répondant à la formule (I) ci-après et obtenus à partir de l'homopolymère de formule $HO-(CHR'CHR''O)_n-R$ où n est un nombre moyen de 2 à 50 et R, $R'$ et $R''$ ont la même signification que ci-dessus :

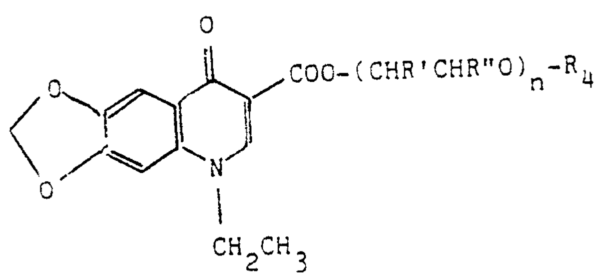

(I)

dans laquelle :

. n, $R'$ et $R''$ ont la même signification que ci-dessus ; et

. $R_4$ représente un atome d'hydrogène, un groupe alkyle en $C_1$-$C_4$ éventuellement substitué par OH, un reste éther cyclique saturé à 5 ou 6 chaînons ou un groupe de formule :

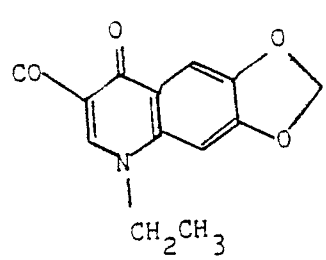

Il est à noter que selon l'invention, on préfère en particulier les dérivés de formule (I) pour lesquels $R' = R'' = H$.

Les composés selon l'invention peuvent aisément être obtenus en condensant l'acide oxolinique ou l'un de ses dérivés réactifs (par exemple le chlorure d'acide de celui-ci), avec l'homopolymère ou le copolymère défini ci-dessus, cette condensation étant de préférence réalisée à chaud dans un solvant organique,

inerte dans les conditions de la réaction, tel que la pyridine ou la DMF par exemple. Selon la quantité et la nature de l'homopolymère ou du copolymère mis en jeu par rapport à l'acide oxolinique, on obtient soit un monoester, mono-thioester ou mono-amide, soit un diester, di-thioester ou di-amide (cas où deux molécules d'acide oxolinique réagissent respectivement avec deux groupes terminaux de l'homopolymère ou du copolymère).

On donne ci-après, à titre d'exemples, le mode de préparation de quelques composés selon l'invention.

Exemple 1 : Préparation du composé (codé JL 93V) de formule (I) de structure particulière :

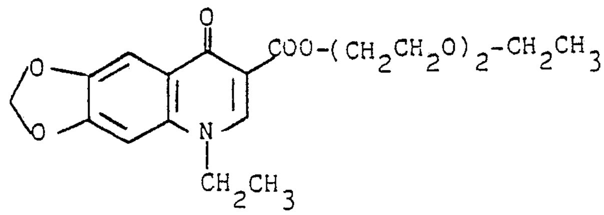

On agite à 100° C pendant 4 heures, un mélange de 5,6 g de chlorure d'acide oxolinique, 3 g d'éthoxyéthoxyéthanol fraîchement distillé et 40 ml de pyridine. On élimine ensuite la pyridine par évaporation sous pression réduite. On dissout le produit obtenu dans du dichlorométhane, lave la phase organique successivement avec HCl (0,1N), $H_2O$, NaOH (0,1N) et $H_2O$, puis la sèche sur $Na_2SO_4$ anhydre et la concentre sous pression réduite. Après addition d'éther et essorage du précipité, on obtient le composé attendu avec un rendement de 85 %.

Exemple 2 : Préparation du composé (codé JL 107 V) de formule (I) et de structure particulière :

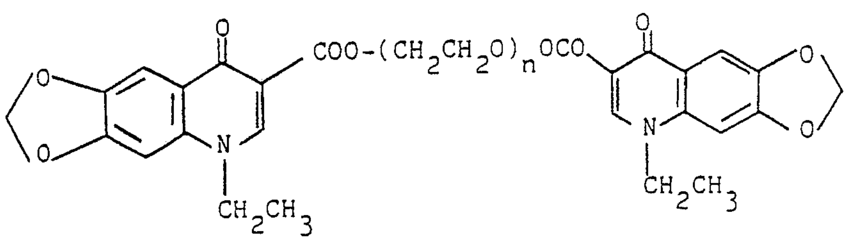

ou n est un nombre moyen égal à 33,7.

On agite à 100° C pendant 24 heures, un mélange de 2 g (7,15 mM) de chlorure d'acide oxolinique, 4,5 g (3 mM) de polyéthylène glycol 1500 et 30 ml de pyridine. On élimine ensuite la pyridine par évaporation sous pression réduite. On dissout le produit obtenu dans du dichlorométhane, lave la phase organique successivement avec HCl (0,1N), $H_2O$, NaOH (0,1N) et $H_2O$, puis la sèche sur $Na_2SO_4$ anhydre et la concentre sous pression réduite. Après addition de méthanol, essorage du précipité formé et séchage de ce dernier dans un dessicateur à 25° C sous 1 mm Hg, on obtient le composé attendu avec un rendement de 50 %.

L'absence d'acide oxolinique libre est vérifiée par chromatographie sur couche mince de silice (éluant = $CH_2Cl_2/CH_3OH$, 9/1 ; révélation à l'iode).

Le taux de substitution S du polyéthylène glycol est déterminé par spectrométrie UV à 254 nm après saponification du produit dans la soude aqueuse 0,1N pendant 2 heures à 80° C.

Par un procédé similaire à celui illustré par les exemples ci-dessus, on obtient d'autres composés selon l'invention et notamment ceux rassemblés dans les tableaux I et II ci-après.

## TABLEAU I

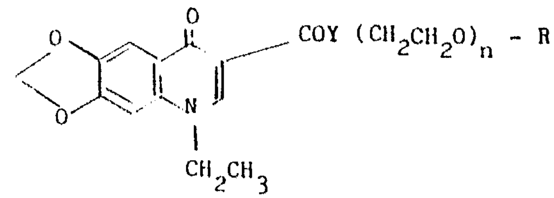

| Composé n° de code | $HY(CH_2CH_2O)_nR$ de départ | | | | S (% en pds) | Point de fusion (°C) | Solubilité | | RMN $^1H$ ($CDCl_3$) δ ppm |
|---|---|---|---|---|---|---|---|---|---|
| | Poids moléculaire | n | R | Y | | | dans l'eau | dans les solvants usuels | |
| JL 93V | 134,2 | 2 | $CH_3CH_2$ | O | Produit pur | 100 - 102 | ± | + | . 1,15, t, J = 7,3 Hz et 1,45, t, J = 7,3 Hz...... 6 H<br>. 3,3 - 4,6, m............ 12 H<br>. 6,0, s ................ 2 H<br>. 6,8, s ................ 1 H<br>. 7,7, s................. 1 H<br>. 8,25, s................ 1 H |
| JL 94V | 164,2 | 3 | $CH_3$ | O | " | 58 - 60 | + | + | . 1,5, t, J = 7,3 Hz....... 3 H<br>. 3,35, s,................ 3 H<br>. 3,5 - 4,6, m,........... 14 H<br>. 6,1, s,................. 2 H<br>. 6,85, s,................ 1 H<br>. 7,75, s,................ 1 H<br>. 8,30, s,................ 1 H |
| JL 95V | 163,2 | 3 | $CH_3$ | NH | " | 120 - 122 | + | + | . 1,5, t, J = 7,3 Hz....... 3 H<br>. 3,3, s,................. 3 H<br>. 3,3 - 3,8, m,........... 12 H<br>. 4,2, q, J = 7,3 Hz....... 2 H<br>. 6,1, s,................. 2 H<br>. 6,85, s,................ 1 H<br>. 7,7, s,................. 1 H<br>. 8,55, s,................ 1 H |

.../...

TABLEAU I (Suite)

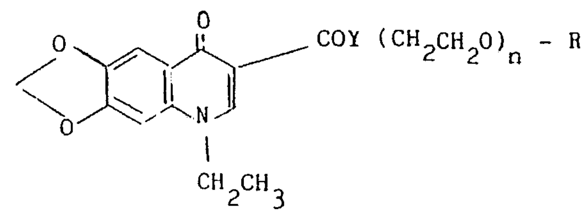

| Composé n° de code | $HY(CH_2CH_2O)_nR$ de départ | | | | S (% en pds) | Point de fusion (°C) | Solubilité | | RMN $^1H$ ($CDCl_3$) δ ppm |
|---|---|---|---|---|---|---|---|---|---|
| | Poids moléculaire | n | R | Y | | | dans l'eau | dans les solvants usuels | |
| JL 103V | ~ 350 | 7,23* | $CH_3$ | O | 80 | - | + | + | - |
| JL 105V | ~1900 | 42,45* | $CH_3$ | O | " | - | + | + | - |
| JL 106V | tétraglycol | 1<n<5 | 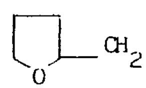 | O | 40 | - | + | + | - |

* nombre moyen

S : déterminé par analyse spectrométrique dans l'U. V. après saponification

TABLEAU II

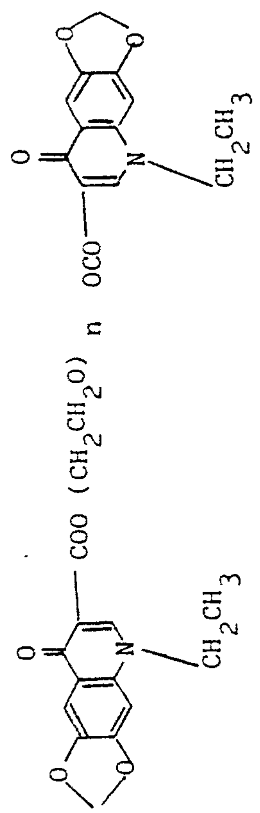

| Composé n° de code | $HO(CH_2CH_2O)_nOH$ de départ: Poids moléculaire | n | S (% en pds) | Solubilité: dans l'eau | dans les solvants usuels |
|---|---|---|---|---|---|
| JL 104V | 300 | 6,41* | 85 | + | + |
| JL 107V | 1 500 | 33,7 * | 63 | + | + |

* nombre moyen

S : déterminé par analyse spectrométrique dans l'U. V. après saponification

Comme on le verra ci-après, les composés selon l'invention possèdent une liaison ester, thioester ou amide facilement hydrolysable dans l'organisme, par voie chimique ou enzymatique, de sorte qu'après coupure de ladite liaison, ils permettent une libération de l'acide oxolinique dans l'organisme et, partant, la conservation de l'activité bactériologique de cet acide tout en modifiant de manière avantageuse la solubilité et les propriétés pharmacologiques de ce dernier.

Ainsi par exemple, l'étude cinétique à température ambiante de l'hydrolyse du composé JL 93V (stable en milieu faiblement acide et en milieu neutre) montre qu'il y a libération d'environ 50 % du principe actif (acide oxolinique) après 24 heures d'hydrolyse en milieu hydroxyde de sodium 0,001 M et coupure immédiate de la liaison ester en milieu hydroxyde de sodium 0,1 M. Cette liaison ester, tout comme d'ailleurs la liaison thioester ou amide, apparaît donc comme étant suffisamment labile par hydrolyse pour

libérer le principe actif dans l'organisme.

On notera en outre que l'acide oxolinique qui est relativement peu soluble dans l'eau ou les solvants organiques habituellement utilisés en galénique, voit sa solubilité augmenter de manière notable lorsqu'il est couplé avec l'homopolymère ou le copolymère. En raison de cette solubilité accrue, il est dès lors possible de concevoir de nouvelles formulations galéniques contenant une teneur plus élevée en acide oxolinique.

Etude de l'activité pharmacocinétique des composés selon l'invention en prenant pour exemple le composé codé JL 93V objet de l'exemple 1 ci-dessus

a/ Détermination des taux plasmatiques après injection par voie intramusculaire chez le chien

Dose administrée : 15 mg/kg en équivalent acide oxolinique
(Comparaison avec l'activité pharmacocinétique de l'acide oxolinique)

TABLEAU III

| Temps (heures) | Taux plasmatiques (en p.p.m.) | |
|---|---|---|
| | Acide oxolinique | JL 93V |
| 0,5 | 6,06 | 7,25 |
| 1 | 6,55 | 16,13 |
| 2 | 5,46 | 15,64 |
| 4 | 3,03 | 10,85 |
| 6 | 2,47 | 6,08 |
| 12 | 1,43 | 2,31 |
| 24 | 0,98 | 0,45 |

Ce tableau III montre que l'on constate des taux plasmatiques plus élevés avec JL 93V qu'avec l'acide oxolinique. De plus, l'aire sous la courbe (taux en fonction du temps) est plus importante pour JL 93V, ce qui permet de conclure à une biodisponibilité accrue ou à un métabolisme différent.

b/ Détermination des taux plasmatiques après injection intraveineuse chez le chien

Dose administrée : 15 mg/kg en équivalent acide oxolinique

TABLEAU IV

| Temps (heures) | Taux plasmatiques (en mcg/ml) | |
|---|---|---|
| | Acide oxolinique | JL 93V |
| 0,5 | 36,79 | 33,62 |
| 1 | 29,81 | 30,21 |
| 2 | 22,32 | 23,35 |
| 6 | 8,89 | 10,94 |
| 8 | 5,65 | 8,23 |
| 10 | 3,59 | 6,35 |
| 24 | 0,15 | 1,19 |

Ce tableau montre qu'il y a dans les deux cas, hydrolyse avec libération très rapide du principe actif avec des taux du même ordre ; toutefois, dans le temps ces taux restent plus élevés pour JL 93V.

c/ Détermination des taux plasmatiques après injection par voie intramusculaire chez le rat

Dose administrée : 20 mg/kg en équivalent acide oxolinique

TABLEAU V

| Temps (heures) | Taux plasmatiques (en p.p.m.) | |
|---|---|---|
| | Acide oxolinique | JL 93V |
| 1 | 0,785 | 15,67 |
| 3 | 0,225 | 8,78 |
| 6 | nul | 3,42 |
| 24 | nul | nul |

On note des taux plus élevés et pendant une durée plus longue pour JL 93V, ainsi qu'une aire sous la courbe plus importante dans le cas de JL 93V.

d/ Détermination de la présence du principe actif dans les tissus après injection par voie intramusculaire chez le rat

Dose administrée : 20 mg/kg en équivalent acide oxolinique

TABLEAU VI

| Temps (heures) | Présence dans les tissus (foie, poumons, reins, muscles) | |
|---|---|---|
| | Acide oxolinique | JL 93V |
| 1 | nulle | positive |
| 3 | nulle | positive |
| 6 | nulle | positive |
| 24 | nulle | nulle |

Ce tableau VI montre que JL 93V conduit à une meilleure imprégnation tissulaire que l'acide oxolinique.

L'ensemble des résultats ci-dessus met en évidence que le couplage de l'acide oxolinique avec un polymère tel que celui défini précédemment, permet une libération retardée de cet acide et, partant, conduit à une durée d'action prolongée ; il permet en outre une distribution et une résorption différentes du principe actif en ce sens que l'on observe des concentrations tissulaires et des taux plasmatiques en principe actif supérieurs à ceux obtenus avec l'acide oxolinique seul.

La toxicité des composés selon l'invention a également été étudiée par la mesure de la dose léthale 50 (DL 50) chez la souris et ils se sont révélés être satisfaisants sur ce plan. A titre d'exemple, on notera que pour le composé JL 93V, la DL 50 (souris ; voie intraveineuse) est de 240 mg/kg et que la DL 50 (souris ; voie orale) est de 1120 mg/kg.

L'étude pharmacologique et toxicologique ci-dessus montre l'intérêt thérapeutique des composés selon l'invention. Ces derniers trouvent donc leur application en tant que médicaments pour l'homme et l'animal, à activité antibiotique et utilisables pour traiter les mêmes affections que celles traitées à ce jour par l'acide oxolinique.

La présente invention s'étend par ailleurs aux compositions pharmaceutiques contenant au moins l'un

des composés selon l'invention, ainsi qu'un ou plusieurs véhicule(s) pharmaceutiquement acceptable(s). Ces compositions peuvent être formulées notamment en vue de leur administration orale et se présenter alors par exemple sous forme de dragées, gélules, comprimés ou solutions buvables, ou en vue de leur administration parentérale et se présenter alors sous la forme de solutions injectables ou encore en vue de leur administration rectales, les méthodes de préparation de ces différentes formes étant bien connues de l'homme de l'art.

Les médicaments et les compositions pharmaceutiques selon l'invention peuvent être administrées, en une ou plusieurs prises, à des doses quotidiennes correspondant à une quantité de principe(s) actif(s) (exprimée en équivalent acide oxolinique) pouvant aller, selon les cas, de 1 à 50 mg/kg.

Exemples de formulations galéniques utilisables par voie orale ou parentérale :

Exemple a : Suspension à 10 % de JL 93V

. JL 93V 10g
. Polysorbate 80 1,3 g
. Parahydroxybenzoate de méthyle 0,120 g
. Parahydroxybenzoate de propyle 0,080 g
. Eau distillée Q.S.P. 100 ml
Dans 80 ml d'eau à ébullition, dissoudre :
Parahydroxybenzoate de méthyle,
Parahydroxybenzoate de propyle.
Refroidir à 20° C, ajouter Polysorbate 80.
Compléter à 100 ml, filtrer sur 0,45 micron, disperser JL 93V et tamiser sur tamis 100 microns.

Exemple b : Solution de JL 93V

. JL 93V 5 g
. Propylène glycol 30 g
. Eau distillée Q.S.P. 100 ml
Dissoudre en chauffant à 40° C le JL 93V dans le Propylène glycol puis compléter à 100 ml avec de l'eau distillgitant.

Exemple c : Solution de JL 94V

. JL 94V 10 g
. Eau distillée Q.S.P. 100 ml
Dissoudre le JL 94V dans 100 ml d'eau distillée.

**Revendications**

1. Ester, thioester ou amide obtenu par réaction entre l'acide oxolinique ou un dérivé réactif de celui-ci au niveau du groupe carboxyle et un groupe terminal OH, SH ou $NH_2$ d'un homopolymère ou un copolymère alterné, séquencé ou statistique, comportant d'une part, une moyenne de 2 à 50 motifs identiques ou différents de structure $-(CHR'CHR''O)-$ où R′ et R″ représentent indépendemment l'un de l'autre un atome d'hydrogène ou un groupe alkyle en $C_1$-$C_4$ et d'autre part, un premier groupe terminal OH, SH ou $NH_2$ et un deuxième groupe terminal OR où R = H, alkyle en $C_1$-$C_4$ éventuellement substitué par OH ou reste éther cyclique saturé à 5 ou 6 chaînons.

2. Composé selon la revendication 1, caractérisé en ce qu'il est obtenu à partir de l'homopolymère de formule $HO-(CHR'CHR''O)_n-R$ où n est un nombre moyen de 2 à 50 et R, R′ et R″ ont la même signification que dans la revendication 1 et en ce qu'il répond à la formule

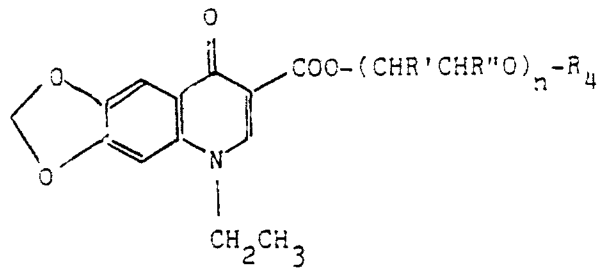

(I)

dans laquelle :
- n, R′ et R″ ont la même signification que ci-dessus ; et
- $R_4$ a la même signification que R dans la revendication 1, mais peut en outre représenter le groupement :

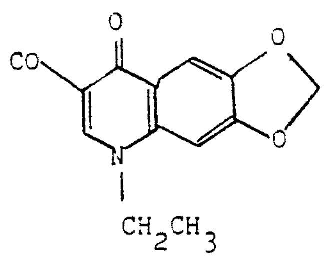

3. Composé selon la revendication 2, caractérisé en ce que R′ = R″ = H.

4. Dérivé de l'acide oxolinique, caractérisé en ce qu'il répond à la formule :

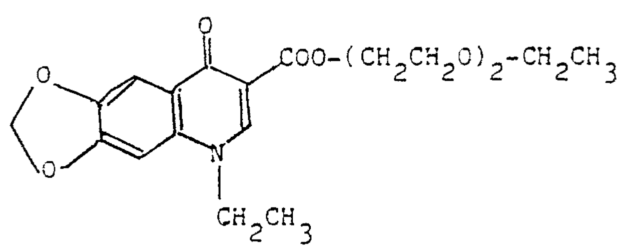

5. Dérivé de l'acide oxolinique, caractérisé en ce qu'il répond à la formule ;

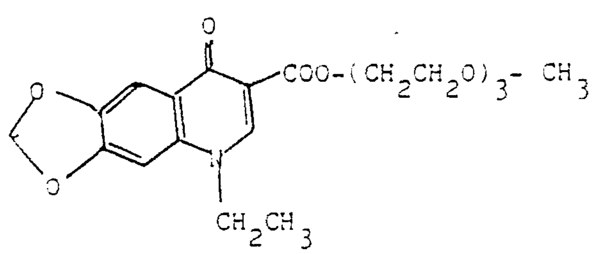

6. Dérivé de l'acide oxolinique, caracterisé en ce qu'il répond à la formule :

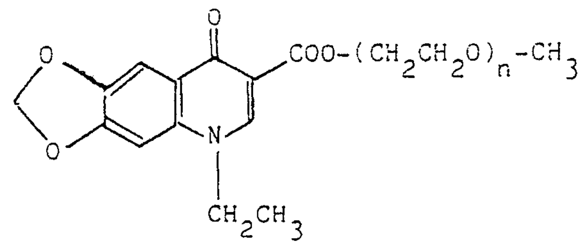

où n est un nombre moyen égal à 7,23 ou 42,45.

7. Dérivé de l'acide oxolinique, caractérisé en ce qu'il répond à la formule :

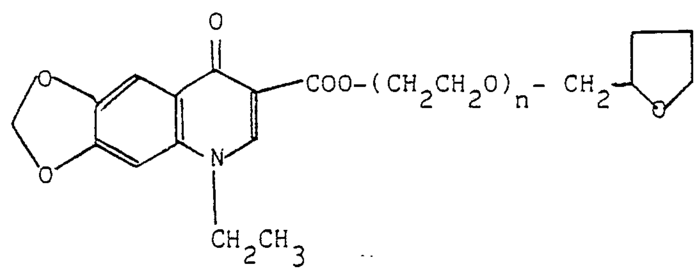

où $1 < n < 5$.

8. Dérivé de l'acide oxolinique, caractérisé en ce qu'il répond à la formule :

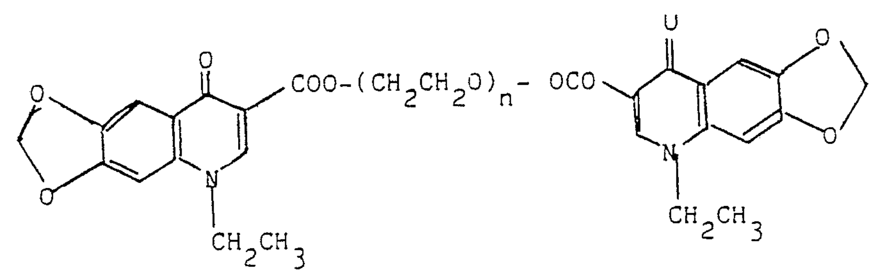

où n est un nombre moyen égal à 6,41 ou 33,7.

9. Procédé de préparation du composé selon la revendication 1, caractérisé en ce qu'il consiste à condenser l'acide oxolinique ou un dérivé réactif de celui-ci au niveau du groupe carboxyle, avec l'homopolymère ou copolymère défini à la revendication 1.

10. Médicament antibiotique, caractérisé en ce qu'il comprend au moins un composé selon l'une des revendication 1 à 8, en association avec un véhicule physiologiquement acceptable.

Revendications pour les Etats Contractants suivants: ES, AT

1. Procédé de préparation d'un ester, thioester ou amide de l'acide oxolinique, caractérisé en ce qu'il consiste à faire réagir l'acide oxolinique ou un dérivé réactif de celui-ci au niveau de son groupe carboxylique, avec un homopolymère ou un compolymère alterné séquencé ou statistique, comportant d'une part une moyenne de 2 à 50, de préférence 2 à 10, motifs identiques ou différents de structure --(CHR′CHR″O)- où R′ et R″ représentent indépendemment l'un de l'autre un atome d'hydrogène ou un

groupe alkyle en $C_1$-$C_4$, et d'autre part, un premier groupe terminal OH, SH ou $NH_2$ et un deuxième groupe terminal OR où R = H, alkyle en $C_1$-$C_4$ éventuellement substitué par un radical hydroxyle, ou reste éther cyclique saturé à 5 ou 6 chaînons.

2. Procédé selon la revendication 1, caractérisé en ce que l'homopolymère répond a la formule HO-$(CHR'CHR''O)_n$-R où n est un nombre moyen de 2 à 50 et R, R' et R'' ont la même signification que dans la revendication 1.

3. Procédé selon la revendication 1 ou 2, caractérisé en ce que l'homopolymère répond à la formule HO-$(CH_2CH_2O)_n$-R.

4. Procédé selon la revendication 3, caractérisé en ce que le couple (n, R) prend la valeur (2, $CH_2CH_3$) ou (3, $CH_3$).

5. Procédé selon la revendication 3, caractérisé en ce que R est méthyle et n est un nombre moyen égal a 7,23 ou 42,45.

6. Procédé selon la revendication 3, caractérisé en ce que n est tel que $1<n<5$ et R possède la formule

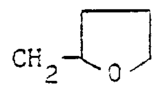

7. Procédé selon la revendication 3, caractérisé en ce que R est un atome d'hydrogène et n est nombre moyen égal a 6,41 ou 33,7.

8. Procédé de préparation d'une composition antibiotique, caractérisé en ce qu'il consiste à adjoindre à l'ester, au thioester ou à l'amide obtenu par le procédé selon l'une des revendications 1 a 7, un véhicule physiologiquement acceptable.

Office européen des brevets

# RAPPORT DE RECHERCHE EUROPEENNE

Numero de la demande

EP 87 40 1722

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | US-A-3 287 458 (D. KAMINSKY et al.)<br>* Colonne 1, lignes 14-65; revendication 3 *<br>--- | 1,10 | C 07 D 491/04<br>C 07 D 519/00<br>C 08 G 65/32<br>A 61 K 31/47<br>A 61 K 31/765<br>A 61 K 47/00 //<br>(C 07 D 491/04<br>C 07 D 317:00<br>C 07 D 221:00 )<br>(C 07 D 519/00<br>C 07 D 491:00<br>C 07 D 491:00 ) |
| Y | EP-A-0 205 029 (BAYER)<br>* Page 14, ligne 1 - page 16, ligne 7; revendications 1,4 *<br>--- | 1,10 | |
| Y | JOURNAL OF MEDICINAL CHEMISTRY, vol. 24, no. 5, mai 1981, pages 622-625, American Chemical Society; R. CECCHI et al.: "Synthesis and pharmacological evaluation of poly(oxyethylene) derivatives of 4-isobutylphenyl-2-propionic acid (ibuprofen)<br>* Page 622, colonne 1, ligne 8 - colonne 2, ligne 4; page 624, "Conclusions" *<br>----- | 1,10 | |
| | | | **DOMAINES TECHNIQUES RECHERCHES (Int. Cl.4)** |
| | | | C 07 D 491/00<br>C 07 D 519/00<br>C 08 G 65/00<br>A 61 K 31/00<br>A 61 K 47/00 |

Le présent rapport a été établi pour toutes les revendications

| Lieu de la recherche | Date d'achèvement de la recherche | Examinateur |
|---|---|---|
| LA HAYE | 14-03-1988 | BRENNAN J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant